# EUROPEAN PATENT APPLICATION

(11) **EP 4 781 920 A1**
(43) Date of publication of application: **29.07.2026**
(21) Application number: 24885278.2
(22) Date of filing: 30.08.2024
(51) Int. Cl.: A61B 10/00, A61B 5/1455, G01N 21/359

(54) **OPTICAL PROBE AND OPTICAL PROBE UNIT**

(30) Priority: 30.10.2023 JP 2023185300
(71) Applicant: Hamamatsu Photonics K.K., Hamamatsu-shi, Shizuoka 435-8558 (JP)
(72) Inventor: SUZUKI, Norihiro, Hamamatsu-shi, Shizuoka 435-8558 (JP); UEDA, Yukio, Hamamatsu-shi, Shizuoka 435-8558 (JP)
(74) Representative: Hoffmann Eitle
(86) International application number: PCT/JP2024/031335
(87) International publication number: WO 2025/094501

(57) **Abstract**

An optical probe comprises: a light reflection unit comprising a reflective surface configured to reflect light, which is incident along a first optical axis, along a second optical axis; a housing that accommodates the light reflection unit such that the light reflection unit is slidable in a first direction parallel to the first optical axis and configured to hold an end of an optical fiber such that the end is positioned on the first optical axis and faces the light reflection unit in the first direction; a first biasing member that biases the light reflection unit to a first side in the first direction in the housing; and an adjustment unit that adjusts a position of the light reflection unit in the first direction by pushing the light reflection unit to a second side in the first direction in the housing.

## Description

### Technical Field

The present disclosure relates to an optical probe and an optical probe unit.

### Background Art

Patent Literature 1 discloses a probe device that is used in a biological optical measurement apparatus for measuring a change in local hemodynamics in a living body. The probe device disclosed in Patent Literature 1 includes a plurality of probes that include a light irradiation probe and a light detection probe, and a shell part that holds the plurality of probes. For each probe, the state of contact between an end of a fiber bundle and the living body can be adjusted in a state where the shell part is mounted on the living body. Accordingly, for example, it is possible to avoid hair or the like being sandwiched between the living body and the end of the fiber bundle and to ensure an optical path between the living body and the probe.

### Citation List

### Patent Literature

[Patent Literature 1] Japanese Unexamined Patent Publication No. 2007-236963

### Summary of Invention

### Technical Problem

However, since the adjustment of the state of contact between the end of the fiber bundle and the living body is performed through the adjustment of the position of the end of the fiber bundle with respect to the living body in the probe device disclosed in Patent Literature 1, there is a concern that a load equal to or larger than an expected load may act on the living body.

Accordingly, an object of the present disclosure is to provide an optical probe and an optical probe unit that can reliably ensure an optical path between a subject and the optical probe while suppressing a load acting on the subject.

### Solution to Problem

An optical probe according to an aspect of the present disclosure is [1] "An optical probe comprising: a light reflection unit comprising a reflective surface configured to reflect light, which is incident along one of a first optical axis and a second optical axis intersecting each other, along the other of the first optical axis and the second optical axis; a housing that accommodates the light reflection unit such that the light reflection unit is slidable in a first direction parallel to the first optical axis and is configured to hold an end of an optical fiber such that the end is positioned on the first optical axis and faces the light reflection unit in the first direction; a first biasing member that biases the light reflection unit to a first side in the first direction in the housing; and an adjustment unit that adjusts a position of the light reflection unit in the first direction by pushing the light reflection unit to a second side opposite to the first side in the first direction in the housing."

The optical probe according to [1] is in a state where the end of the optical fiber faces the light reflection unit on the first optical axis and a subject faces the light reflection unit on the second optical axis. In this state, the first biasing member biases the light reflection unit, which is slidable in the first direction, to the first side in the first direction, but the adjustment unit pushes the light reflection unit, which is slidable in the first direction, to the second side in the first direction. As a result, the position of the light reflection unit in the first direction can be adjusted. Therefore, for example, it is possible to avoid hair or the like being sandwiched between the subject and the light reflection unit and to ensure an optical path between the subject and the light reflection unit. Here, the sliding direction of the light reflection unit, the biasing direction of the first biasing member, and the pushing direction caused by the adjustment unit are all the first direction parallel to the first optical axis and intersect a direction parallel to the second optical axis on which the subject faces the light reflection unit. Therefore, in a case where the position of the light reflection unit in the first direction is to be adjusted, it is possible to suppress a load acting on the subject. As described above, according to the optical probe of [1], it is possible to reliably ensure an optical path between the optical probe and the subject while suppressing the load acting on the subject.

An optical probe according to an aspect of the present disclosure is [2] "The optical probe according to [1], in which the light reflection unit comprises an inclined surface corresponding to the reflective surface, the adjustment unit comprises a pushing member comprising a pushing surface that is in contact with the inclined surface, and the housing accommodates the pushing member such that the pushing member is slidable in a second direction parallel to the second optical axis". According to the optical probe of [2], by using the inclined surface of the light reflection unit corresponding to the reflective surface to adjust the position of the light reflection unit in the first direction, the operation of the adjustment unit can be easily and reliably performed from a side opposite to the subject in the second direction.

An optical probe according to an aspect of the present disclosure is [3] "The optical probe according to [2], in which the adjustment unit further comprises a screw member that is attached to the housing to be movable forward and backward along the second direction, one end of the screw member in the second direction is in contact with the pushing member, and the other end of the screw member in the second direction is exposed to the outside of the housing". According to the optical probe of [3], it is possible to more easily and more reliably perform the operation of the adjustment unit from a side opposite to the subject in the second direction through access to the other end of the screw member in the second direction.

An optical probe according to an aspect of the present disclosure is [4] "The optical probe according to [3], in which a recessed portion in which the one end of the screw member is disposed is formed in the pushing member". According to the optical probe of [4], the misalignment of one end of the screw member relative to the pushing member can be suppressed, and the position of the light reflection unit in the first direction can be adjusted with high accuracy.

An optical probe according to an aspect of the present disclosure is [5] "The optical probe according to any one of [2] to [4], in which the light reflection unit comprises a light transmission member and a metal layer that is disposed on a surface of the light transmission member, and the reflective surface is a surface of the metal layer facing the light transmission member". According to the optical probe of [5], light incident along one of the first optical axis and the second optical axis can be reliably reflected along the other of the first optical axis and the second optical axis.

An optical probe according to an aspect of the present disclosure is [6] "The optical probe according to [5], in which the light reflection unit further comprises a protective layer that is disposed on a surface of the metal layer opposite to the light transmission member, and the inclined surface is a surface of the protective layer opposite to the metal layer". According to the optical probe of [6], it is possible to set the state of the contact between the inclined surface of the light reflection unit and the pushing surface of the pushing member to a desired state while suppressing the abrasion of the metal layer.

An optical probe according to an aspect of the present disclosure is [7] "The optical probe according to any one of [2] to [4], in which the light reflection unit comprises a light transmission member, and the light transmission member comprises the reflective surface and the inclined surface as the same surface". According to the optical probe of [7], reflecting light, which is incident along one of the first optical axis and the second optical axis, along the other of the first optical axis and the second optical axis can be realized with a simple configuration.

An optical probe according to an aspect of the present disclosure is [8] "The optical probe according to [7], in which the pushing surface is a rough surface". According to the optical probe of [8], a sufficient difference in refractive index can be ensured on the reflective surface of the light transmission member, so that light incident along one of the first optical axis and the second optical axis can be reliably reflected along the other of the first optical axis and the second optical axis.

An optical probe according to an aspect of the present disclosure is [9] "The optical probe according to any one of [1] to [8], further comprising: a second biasing member that biases the light reflection unit to the second side in the first direction in the housing". According to the optical probe of [9], the adjustment unit pushes the light reflection unit to the second side in the first direction, so that the light reflection unit can be caused to smoothly and stably slide to the position where the adjustment unit positions the light reflection unit.

An optical probe according to an aspect of the present disclosure is [10] "The optical probe according to [9], in which a biasing force of the first biasing member is larger than a biasing force of the second biasing member". According to the optical probe of [10], the adjustment unit pushes the light reflection unit to the second side in the first direction, so that the light reflection unit can be caused to more smoothly and more stably slide to the position where the adjustment unit positions the light reflection unit.

An optical probe according to an aspect of the present disclosure is [11] "The optical probe according to [1], in which the adjustment unit further comprises a screw member that is attached to the housing to be movable forward and backward along the first direction, one end of the screw member in the first direction is in contact with the light reflection unit, and the other end of the screw member in the first direction is exposed to the outside of the housing". According to the optical probe of [11], the adjustment of the position of the light reflection unit in the first direction can be realized with a simple configuration.

An optical probe unit according to an aspect of the present disclosure is [12] "An optical probe unit comprising: a plurality of optical probes, each of which is the optical probe according to any one of [1] to [11]; and a holder that holds the plurality of optical probes".

According to the optical probe unit of [12], the plurality of optical probes are used as an optical probe for guiding light with which the subject is irradiated and as an optical probe for guiding light emitted from the subject. Accordingly, it is possible to reliably ensure an optical path between the plurality of optical probes and the subject while suppressing the load acting on the subject. As a result, the optical examination of the subject can be performed with high accuracy.

### Advantageous Effects of Invention

According to the present disclosure, it is possible to provide an optical probe and an optical probe unit that can reliably ensure an optical path between a subject and the optical probe while suppressing a load acting on the subject.

### Brief Description of Drawings

FIG. 1 is a configuration diagram of an optical measurement device including an optical probe unit according to an embodiment.
FIG. 2 is a plan view of an optical probe included in the optical probe unit shown in FIG. 1.
FIG. 3 is a cross-sectional view of the optical probe taken along line III-III shown in FIG. 2.
FIG. 4 is a cross-sectional view of the optical probe taken along line IV-IV shown in FIG. 2.
FIG. 5 is a cross-sectional view of the optical probe taken along line III-III shown in FIG. 2.

### Description of Embodiments

Hereinafter, an embodiment of the present disclosure will be described in detail with reference to drawings. In the following description, the same or corresponding elements will be denoted by the same reference numerals, and redundant descriptions will be omitted.

### [Configuration of Optical Measurement Device]

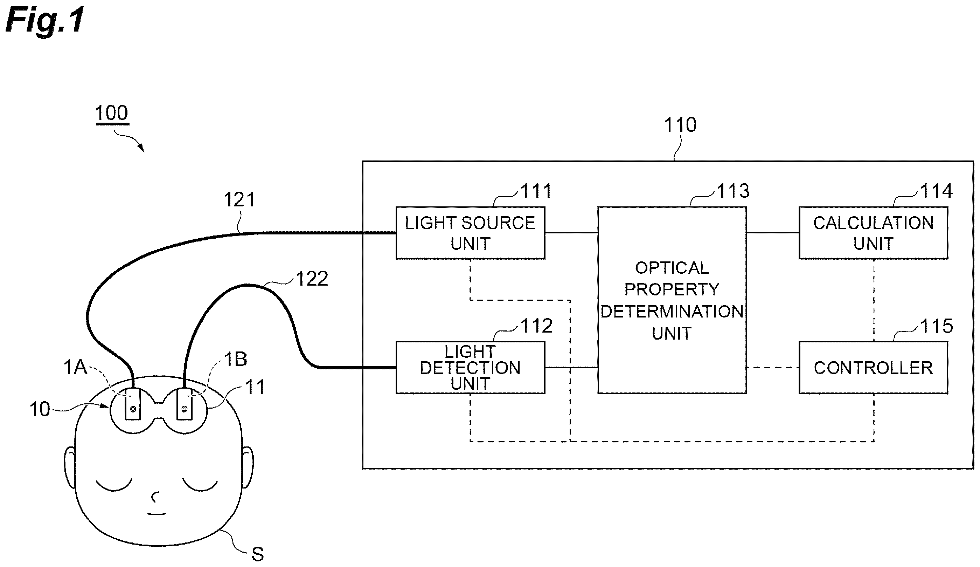

As shown in FIG. 1, an optical measurement device 100 includes an optical probe unit 10 and a device body 110. The optical measurement device 100 irradiates a predetermined site of a subject S with light and detects light emitted from the predetermined site of the subject S via the optical probe unit 10, which is mounted on the subject S, to measure optical properties at the predetermined site of the subject S. For example, the optical measurement device 100 irradiates a deep brain portion with near-infrared light and detects light emitted from the deep brain portion via the optical probe unit 10, which is mounted on the head of a person who is the subject S, to measure the amount of light attenuated or the amount of light diffused with time at the deep brain portion, and measures hemoglobin dynamics (for example, oxygenated hemoglobin concentration, decarbonized hemoglobin concentration, and tissue oxygen saturation) at the deep brain portion based on the measured values. Further, the subject S that is an object on which the optical probe unit 10 is to be mounted may be a site other than the head of a person, or furthermore, may be a site of a living body other than a person.

The optical probe unit 10 includes a pair of optical probes 1A and 1B and a holder 11. The optical probe 1A is a probe that emits light to the subject S, and the optical probe 1B is a probe on which light is incident from the subject S. The holder 11 holds the pair of optical probes 1A and 1B. Specifically, the holder 11 holds the pair of optical probes 1A and 1B such that a distance between the pair of optical probes 1A and 1B is maintained at a predetermined distance and a light-emitting surface of the optical probe 1A and a light incident surface of the optical probe 1B are in contact with the subject S. The holder 11 is, for example, a pad-like member having elasticity and flexibility.

The device body 110 includes a light source unit 111, a light detection unit 112, an optical property determination unit 113, a calculation unit 114, and a controller 115. The light source unit 111 is optically connected to the optical probe 1A via an optical fiber 121. The light detection unit 112 is optically connected to the optical probe 1B via an optical fiber 122.

The light source unit 111 emits light with which the predetermined site of the subject S is to be irradiated. The light source unit 111 is formed of a light-emitting element (for example, a light-emitting diode, a laser diode, or the like). The light emitted from the light source unit 111 is guided from the light source unit 111 to the optical probe 1A by the optical fiber 121, and the predetermined site of the subject S is irradiated with the light from the optical probe 1A.

The light detection unit 112 detects the light emitted from the predetermined site of the subject S. The light detection unit 112 is formed of a light detection element (for example, a photomultiplier tube, a photodiode, an avalanche photodiode, a PIN photodiode, a Multi-Pixel Photon Counter (MPPC), or the like). The light emitted from the predetermined site of the subject S is guided from the optical probe 1A to the light detection unit 112 by the optical fiber 122, and is detected by the light detection unit 112.

The optical property determination unit 113 determines optical properties at the predetermined site of the subject S based on a detection signal output from the light detection unit 112. The calculation unit 114 derives a measured value at the predetermined site of the subject S based on optical property data output from the optical property determination unit 113. The controller 115 controls the light source unit 111, the light detection unit 112, the optical property determination unit 113, and the calculation unit 114. For example, in the optical measurement device 100, the hemoglobin dynamics at the deep brain portion of the subject S are derived by the optical property determination unit 113 and the calculation unit 114.

### [Configuration of Optical Probe]

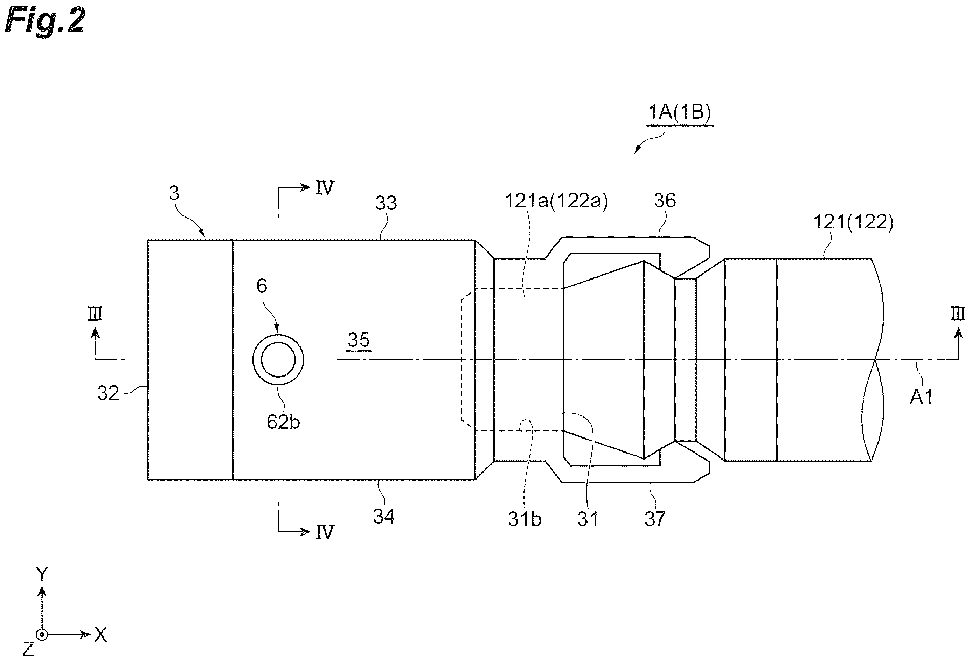

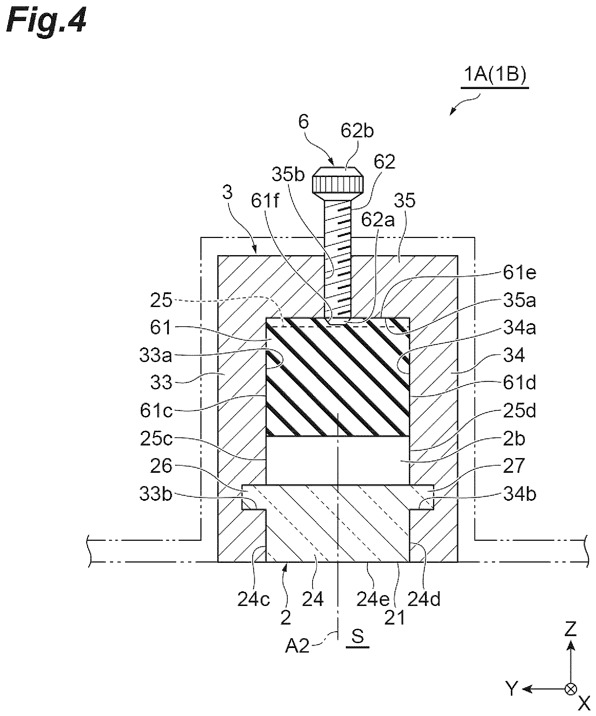

As shown in FIGS. 2, 3, and 4, each of the optical probes 1A and 1B includes a light reflection unit 2, a housing 3, a first biasing member 4, a second biasing member 5, and an adjustment unit 6. The optical probe 1A emits light, which is incident from an end 121a of the optical fiber 121 along a first optical axis A1, to the subject S along a second optical axis A2. The optical probe 1B emits light, which is incident from the subject S along the second optical axis A2, to an end 122a of the optical fiber 122 along the first optical axis A1. Since the optical probe 1B has the same configuration as the optical probe 1A, the configuration of the optical probe 1A will be described below and the description of the configuration of the optical probe 1B will be omitted. In the following description, a first direction parallel to the first optical axis A1 will be referred to as an X-axis direction, a second direction parallel to the second optical axis A2 will be referred to as a Z-axis direction, and a direction perpendicular to the first optical axis A1 and the second optical axis A2 will be referred to as a Y-axis direction. The second optical axis A2 is orthogonal to the first optical axis A1 in the present embodiment, but only needs to intersect the first optical axis A1.

The light reflection unit 2 includes a light transmission member 21, a metal layer 22, and a protective layer 23. The light transmission member 21 includes a support portion 24, a reflective portion 25, and a pair of protruding portions 26 and 27. The reflective portion 25 is disposed on one side of the support portion 24 in the Z-axis direction. The pair of protruding portions 26 and 27 are disposed on both sides of the support portion 24 in the Y-axis direction. For example, the support portion 24, the reflective portion 25, and the pair of protruding portions 26 and 27 are integrally formed of a transparent resin.

The support portion 24 has a plurality of surfaces 24a, 24b, 24c, 24d, and 24e. The surface 24a is a surface perpendicular to the X-axis direction, and is a surface facing one side in the X-axis direction. The surface 24b is a surface perpendicular to the X-axis direction, and is a surface facing the other side in the X-axis direction. The surface 24c is a surface perpendicular to the Y-axis direction, and is a surface facing one side in the Y-axis direction. The surface 24d is a surface perpendicular to the Y-axis direction, and is a surface facing the other side in the Y-axis direction. The surface 24e is a surface perpendicular to the Z-axis direction, and is a surface orthogonal to the second optical axis A2.

The reflective portion 25 has a plurality of surfaces 25a, 25b, 25c, and 25d. The surface 25a is a surface perpendicular to the X-axis direction, and is a surface orthogonal to the first optical axis A1. The surface 25b is a surface parallel to the Y-axis direction, and is a surface on which the first optical axis A1 and the second optical axis A2 intersect each other in a relationship of an incident angle and a reflection angle. The surface 25c is a surface perpendicular to the Y-axis direction, and is a surface facing one side in the Y-axis direction. The surface 25d is a surface perpendicular to the Y-axis direction, and is a surface facing the other side in the Y-axis direction.

The protruding portion 26 protrudes from the surface 24c of the support portion 24 such that a height direction thereof is parallel to the Y-axis direction, and extends in the X-axis direction. The protruding portion 27 protrudes from the surface 24d of the support portion 24 such that a height direction thereof is parallel to the Y-axis direction, and extends in the X-axis direction.

The surface 24a of the support portion 24 and the surface 25a of the reflective portion 25 are positioned on the same plane. The surface 24c of the support portion 24 and the surface 25c of the reflective portion 25 are positioned on the same plane. The surface 24d of the support portion 24 and the surface 25d of the reflective portion 25 are positioned on the same plane.

The metal layer 22 is disposed on the surface 25b of the reflective portion 25, which is the surface of the light transmission member 21. In the light reflection unit 2, a surface of the metal layer 22 facing the light transmission member 21 is a reflective surface 2a reflecting light, which is incident along the first optical axis A1, along the second optical axis A2. The material of the metal layer 22 is, for example, aluminum, silver, vanadium, or gold. The thickness of the metal layer 22 is, for example, 0.7 µm or more and 0.8 µm or less. In the optical probe 1B, the reflective surface 2a reflects light, which is incident along the second optical axis A2, along the first optical axis A1.

The protective layer 23 is disposed on a surface of the metal layer 22 opposite to the light transmission member 21. In the light reflection unit 2, a surface of the protective layer 23 opposite to the metal layer 22 is an inclined surface 2b corresponding to the reflective surface 2a. The material of the protective layer 23 is, for example, silicon monoxide (SiO). The thickness of the protective layer 23 is, for example, 1 nm or more and 0.1 µm or less. The fact that the inclined surface 2b corresponds to the reflective surface 2a means that the inclined surface 2b faces the reflective surface 2a via at least one layer or that the inclined surface 2b is the same surface as the reflective surface 2a.

The housing 3 includes a plurality of wall portions 31, 32, 33, 34, and 35 and a pair of claw portions 36 and 37. The wall portion 31 is disposed on one side of an internal space of the housing 3 in the X-axis direction. The wall portion 31 has an inner surface 31a perpendicular to the X-axis direction. The wall portion 32 is disposed on the other side of the internal space of the housing 3 in the X-axis direction. The wall portion 32 has an inner surface 32a perpendicular to the X-axis direction. The wall portion 33 is disposed on one side of the internal space of the housing 3 in the Y-axis direction. The wall portion 33 has an inner surface 33a perpendicular to the Y-axis direction. The wall portion 34 is disposed on the other side of the internal space of the housing 3 in the Y-axis direction. The wall portion 34 has an inner surface 34a perpendicular to the Y-axis direction. The wall portion 35 is disposed on one side of the internal space of the housing 3 in the Z-axis direction. The wall portion 35 has an inner surface 35a perpendicular to the Z-axis direction. The pair of claw portions 36 and 37 face each other in the Y-axis direction. Each of the claw portions 36 and 37 extends from the wall portion 31 to a side opposite to the internal space of the housing 3. For example, the plurality of wall portions 31, 33, 34, and 35 and the pair of claw portions 36 and 37 are integrally formed of a black resin, and the wall portion 32 is formed of a black resin to be separate from the wall portions 31, 33, 34, and 35 and the pair of claw portions 36 and 37.

A groove 33b is formed in the wall portion 33. The groove 33b is open to the inner surface 33a such that a depth direction thereof is parallel to the Y-axis direction, and extends in the X-axis direction. A groove 34b is formed in the wall portion 34. The groove 34b is open to the inner surface 34a such that a depth direction thereof is parallel to the Y-axis direction, and extends in the X-axis direction. The light reflection unit 2 is disposed in the housing 3 in a state where the protruding portion 26 is disposed in the groove 33b and the protruding portion 27 is disposed in the groove 34b. A distance between the surface 24a of the support portion 24 and the surface 24b of the support portion 24 in the X-axis direction (that is, a distance between the surface 25a of the reflective portion 25 and the surface 24b of the support portion 24 in the X-axis direction) is shorter than a distance between the inner surface 31a of the wall portion 31 and the inner surface 32a of the wall portion 32 in the X-axis direction. The surface 24c of the support portion 24 and the surface 25c of the reflective portion 25 are in contact with the inner surface 33a of the wall portion 33. The surface 24d of the support portion 24 and the surface 25d of the reflective portion 25 are in contact with the inner surface 34a of the wall portion 34. Accordingly, the light reflection unit 2 is slidable in the X-axis direction in the housing 3. That is, the housing 3 accommodates the light reflection unit 2 such that the light reflection unit 2 is slidable in the X-axis direction. End surfaces of the respective wall portions 31, 32, 33, and 34 opposite to the wall portion 35 and the surface 24e of the support portion 24 are positioned on the same plane.

A through-hole 31b is formed in the wall portion 31. The through-hole 31b penetrates the wall portion 31 such that a center line thereof coincides with the first optical axis A1. The through-hole 31b faces the surface 25a of the reflective portion 25 in the X-axis direction. The through-hole 31b is positioned between the pair of claw portions 36 and 37 as viewed in the X-axis direction. A part of the end 121a of the optical fiber 121 is disposed in the through-hole 31b. Accordingly, the end 121a of the optical fiber 121 faces the light reflection unit 2 on the first optical axis A1. In this state, the end 121a of the optical fiber 121 is gripped by the pair of claw portions 36 and 37. That is, the housing 3 holds the end 121a of the optical fiber 121 such that the end 121a is positioned on the first optical axis A1 and faces the light reflection unit 2 in the X-axis direction.

A bottomed hole 31c is formed in the wall portion 31. The bottomed hole 31c is open to the inner surface 31a of the wall portion 31 such that a depth direction thereof is parallel to the X-axis direction. The bottomed hole 31c faces the surface 24a of the support portion 24 in the X-axis direction. The first biasing member 4 is disposed between the bottom surface of the bottomed hole 31c and the surface 24a of the support portion 24. The first biasing member 4 is a coil spring that is disposed in a compressed state between the bottom surface of the bottomed hole 31c and the surface 24a of the support portion 24.

A bottomed hole 32b is formed in the wall portion 32. The bottomed hole 32b is open to the inner surface 32a of the wall portion 32 such that a depth direction thereof is parallel to the X-axis direction. The bottomed hole 32b faces the surface 24b of the support portion 24 in the X-axis direction. The second biasing member 5 is disposed between the bottom surface of the bottomed hole 32b and the surface 24b of the support portion 24. The second biasing member 5 is a coil spring that is disposed in a compressed state between the bottom surface of the bottomed hole 32b and the surface 24b of the support portion 24.

The first biasing member 4 biases the light reflection unit 2 to an X1 side (first side) in the X-axis direction in the housing 3. The second biasing member 5 biases the light reflection unit 2 to an X2 side (a second side opposite to the first side) in the X-axis direction in the housing 3. The biasing force of the first biasing member 4 is larger than the biasing force of the second biasing member 5.

The adjustment unit 6 pushes the light reflection unit 2 to the X2 side in the X-axis direction in the housing 3 to adjust the position of the light reflection unit 2 in the X-axis direction. In the present embodiment, the adjustment unit 6 includes a pushing member 61 and a screw member 62.

The pushing member 61 has a pushing surface 61a. The pushing surface 61a is in contact with the inclined surface 2b of the light reflection unit 2. More specifically, the pushing surface 61a is a surface parallel to the inclined surface 2b of the light reflection unit 2, and is in surface contact with the inclined surface 2b of the light reflection unit 2. The pushing member 61 further has a plurality of surfaces 61b, 61c, 61d, and 61e. The surface 61b is a surface perpendicular to the X-axis direction, and is in contact with the inner surface 32a of the wall portion 32. The surface 61c is a surface perpendicular to the Y-axis direction, and is in contact with the inner surface 33a of the wall portion 33. The surface 61d is a surface perpendicular to the Y-axis direction, and is in contact with the inner surface 34a of the wall portion 34. The surface 61e is a surface perpendicular to the Z-axis direction, and faces the inner surface 35a of the wall portion 35. Accordingly, the pushing member 61 is slidable in the Z-axis direction in the housing 3. That is, the housing 3 accommodates the pushing member 61 such that the pushing member 61 is slidable in the Z-axis direction. For example, the pushing member 61 is formed of a black resin.

A recessed portion 61f is formed in the pushing member 61. The recessed portion 61f is open to the surface 61e of the pushing member 61 such that a depth direction thereof is parallel to the Z-axis direction. A screw hole 35b is formed in the wall portion 35. The screw hole 35b penetrates the wall portion 35 in the Z-axis direction. The screw hole 35b faces the recessed portion 61f in the Z-axis direction. The screw member 62 is threadedly engaged with the screw hole 35b and penetrates the wall portion 35 along the Z-axis direction. That is, the screw member 62 is attached to the housing 3 to be movable forward and backward along the Z-axis direction. One end 62a of the screw member 62 in the Z-axis direction is disposed in the recessed portion 61f, and is in contact with the pushing member 61. The other end 62b of the screw member 62 in the Z-axis direction is exposed to the outside of the housing 3. The other end 62b of the screw member 62 is configured as a knob for rotation.

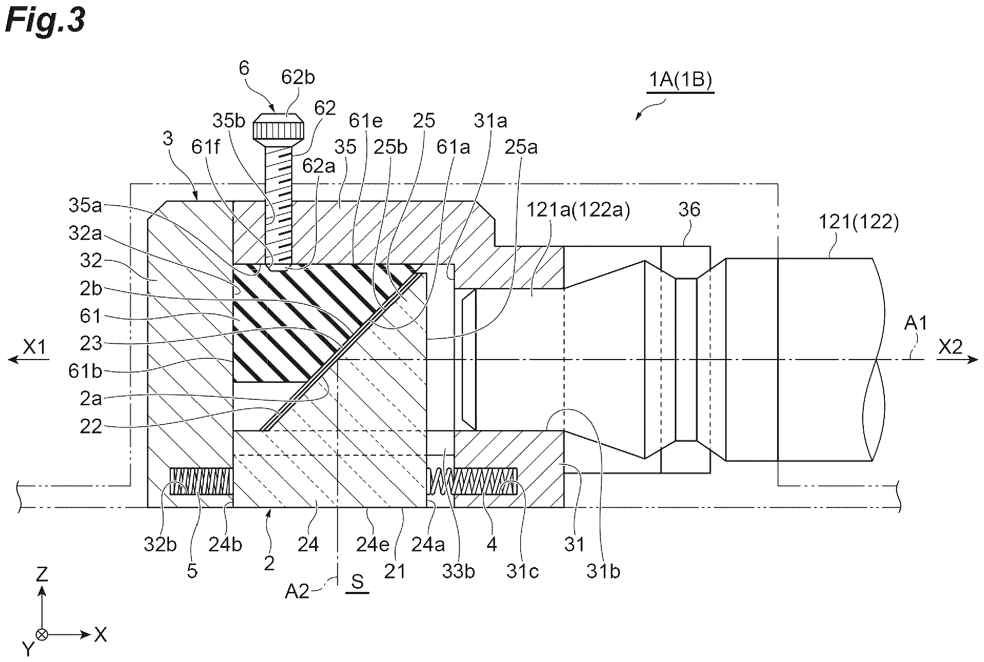

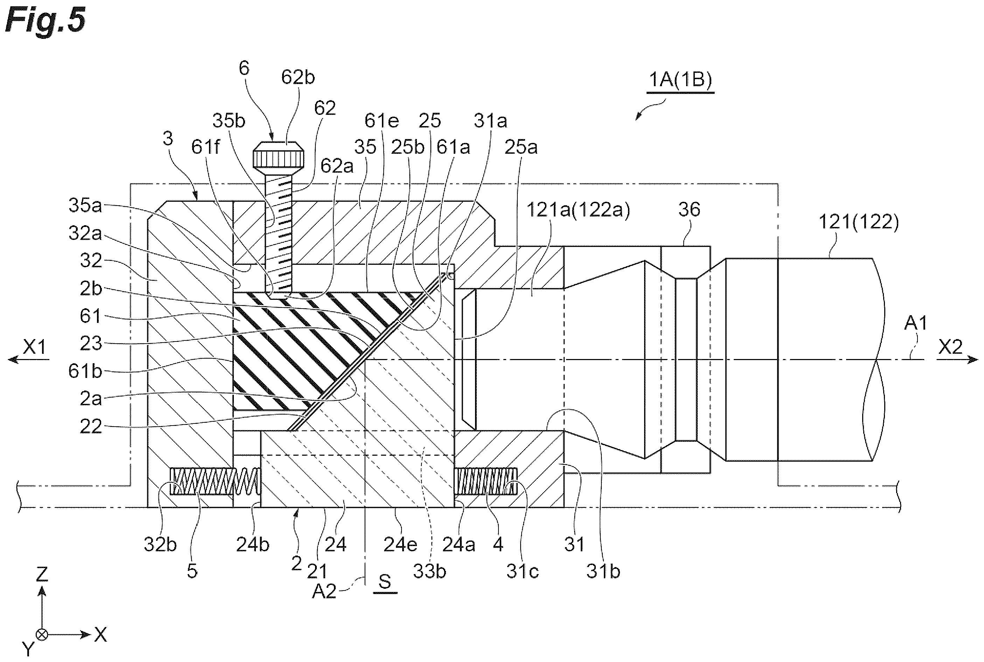

In the optical probe 1A configured as described above, in a case where the other end 62b of the screw member 62 is rotated in a normal direction and the screw member 62 is moved forward such that a state shown in FIG. 3 is changed to a state shown in FIG. 5, the pushing member 61 slides to a side opposite to the wall portion 35 due to the pushing force of the screw member 62 and the inclined surface 2b of the light reflection unit 2 is pushed by the pushing surface 61a of the pushing member 61. As a result, the light reflection unit 2 slides to the X2 side. On the other hand, in a case where the other end 62b of the screw member 62 is rotated in a reverse direction and the screw member 62 is moved backward such that the state shown in FIG. 5 is changed to the state shown in FIG. 3, the light reflection unit 2 slides to the X1 side due to a biasing force obtained by subtracting the biasing force of the second biasing member 5 from the biasing force of the first biasing member 4 and the pushing surface 61a of the pushing member 61 is pushed by the inclined surface 2b of the light reflection unit 2. As a result, the pushing member 61 slides to the wall portion 35. As described above, in the optical probe 1A, the position of the light reflection unit 2 in the X-axis direction (that is, the position of the second optical axis A2) is adjusted as the other end 62b of the screw member 62 is rotated.

### [Operation and Effects]

The optical probe 1A (1B) is in a state where the end 121a (122a) of the optical fiber 121 (122) faces the light reflection unit 2 on the first optical axis A1 and the subject S faces the light reflection unit 2 on the second optical axis A2. In this state, the first biasing member 4 biases the light reflection unit 2, which is slidable in the X-axis direction, to the X1 side in the X-axis direction, but the adjustment unit 6 pushes the light reflection unit 2, which is slidable in the X-axis direction, to the X2 side in the X-axis direction. As a result, the position of the light reflection unit 2 in the X-axis direction can be adjusted. Therefore, for example, it is possible to avoid hair or the like being sandwiched between the subject S and the light reflection unit 2 and to ensure an optical path between the subject S and the light reflection unit 2. Here, the sliding direction of the light reflection unit 2, the biasing direction of the first biasing member 4, and the pushing direction caused by the adjustment unit 6 are all the X-axis direction parallel to the first optical axis A1 and intersect a direction parallel to the second optical axis A2 on which the subject S faces the light reflection unit 2. Therefore, in a case where the position of the light reflection unit 2 in the X-axis direction is to be adjusted, it is possible to suppress a load acting on the subject S. As described above, according to the optical probe 1A (1B), it is possible to reliably ensure an optical path between the optical probe and the subject S while suppressing the load acting on the subject S.

In particular, in a case where the head of an infant is the subject S, the head is immature both functionally and structurally. Further, in order to minimize the variation in respiratory and circulatory dynamics, it is required to minimize the time for which the optical probe unit 10 is mounted on the subject S. On the other hand, according to the optical probe 1A (1B), as described above, it is possible to reliably ensure an optical path between the optical probe and the subject S while suppressing the load acting on the subject S.

In the optical probe 1A (1B), the light reflection unit 2 has the inclined surface 2b corresponding to the reflective surface 2a, the adjustment unit 6 includes the pushing member 61 having the pushing surface 61a that is in contact with the inclined surface 2b, and the housing 3 accommodates the pushing member 61 such that the pushing member 61 is slidable in the Z-axis direction parallel to the second optical axis A2. Accordingly, by using the inclined surface 2b of the light reflection unit 2, which corresponds to the reflective surface 2a, for adjustment of the position of the light reflection unit 2 in the X-axis direction, the operation of the adjustment unit 6 can be easily and reliably performed from a side opposite to the subject S in the Z-axis direction. Further, since the adjustment unit 6 can be operated at a position away from the subject S, it is possible to suppress the contact between the subject S and an instrument and a hand that are used to operate the adjustment unit 6.

In the optical probe 1A (1B), the adjustment unit 6 includes the screw member 62 that is attached to the housing 3 to be movable forward and backward along the Z-axis direction, one end 62a of the screw member 62 in the Z-axis direction is in contact with the pushing member 61, and the other end 62b of the screw member 62 in the Z-axis direction is exposed to the outside of the housing 3. Accordingly, it is possible to more easily and more reliably perform the operation of the adjustment unit 6 from a side opposite to the subject S in the Z-axis direction through access to the other end 62b of the screw member 62 in the Z-axis direction.

In the optical probe 1A (1B), the recessed portion 61f in which one end 62a of the screw member 62 is disposed is formed in the pushing member 61. Accordingly, the misalignment of one end 62a of the screw member 62 relative to the pushing member 61 can be suppressed, and the position of the light reflection unit 2 in the X-axis direction can be adjusted with high accuracy.

In the optical probe 1A (1B), the light reflection unit 2 includes the metal layer 22 disposed on the surface of the light transmission member 21, and the surface of the metal layer 22 facing the light transmission member 21 is the reflective surface 2a. Accordingly, light incident along one of the first optical axis A1 and the second optical axis A2 can be reliably reflected along the other of the first optical axis A1 and the second optical axis A2.

In the optical probe 1A (1B), the light reflection unit 2 includes the protective layer 23 that is disposed on the surface of the metal layer 22 opposite to the light transmission member 21, and the surface of the protective layer 23 opposite to the metal layer 22 is the inclined surface 2b. Accordingly, it is possible to set the state of the contact between the inclined surface 2b of the light reflection unit 2 and the pushing surface 61a of the pushing member 61 to a desired state while suppressing the abrasion of the metal layer 22.

In the optical probe 1A (1B), the second biasing member 5 biases the light reflection unit 2 to the X2 side in the X-axis direction in the housing 3. Accordingly, the adjustment unit 6 pushes the light reflection unit 2 to the X2 side in the X-axis direction, so that the light reflection unit 2 can be caused to smoothly and stably slide to the position where the adjustment unit 6 positions the light reflection unit 2.

In the optical probe 1A (1B), the biasing force of the first biasing member 4 is larger than the biasing force of the second biasing member 5. Accordingly, the adjustment unit 6 pushes the light reflection unit 2 to the X2 side in the X-axis direction, so that the light reflection unit 2 can be caused to more smoothly and more stably slide to the position where the adjustment unit 6 positions the light reflection unit 2.

In the optical probe 1A (1B), the light reflection unit 2 includes the support portion 24 and the reflective portion 25 that are arranged in the Z-axis direction, and the support portion 24 is biased to the X1 side in the X-axis direction by the first biasing member 4. Then, the inclined surface 2b provided on the surface 25a of the reflective portion 25 is pushed to the X2 side in the X-axis direction by the adjustment unit 6. The reflective surface 2a provided on the surface 25a of the reflective portion 25 guides light from one of the first optical axis A1 and the second optical axis A2 to the other of the first optical axis A1 and the second optical axis A2. Accordingly, it is possible to simultaneously achieve biasing the light transmission member 21 to the X1 side, pushing the light transmission member 21 to the X2 side, and guiding light from one of the first optical axis A1 and the second optical axis A2 to the other of the first optical axis A1 and the second optical axis A2.

According to the optical probe unit 10, the optical probe 1A guides light with which the subject S is to be irradiated, and the optical probe 1B guides light emitted from the subject S. Accordingly, it is possible to reliably ensure an optical path between the pair of optical probes 1A and 1B and the subject S while suppressing the load acting on the subject S. As a result, the optical examination of the subject S can be performed with high accuracy.

### [Modification Examples]

The present disclosure is not limited to the above-described embodiment. The pushing member 61 is slidable in the Z-axis direction parallel to the second optical axis A2 in the above-described embodiment, but the present disclosure is not limited thereto. For example, the pushing member 61 may be slidable in a direction that is inclined with respect to the second optical axis A2 at an angle of 45 degrees or less. Even in this case, the light reflection unit 2 can slide in the X-axis direction due to the sliding of the pushing member 61.

The inclined surface 2b faces the reflective surface 2a via at least one layer in the above-described embodiment, but the present invention is not limited thereto. In an optical probe 1A (1B) of a first modification example, the light transmission member 21 may have the reflective surface 2a and the inclined surface 2b as the same surface. That is, the inclined surface 2b only needs to correspond to the reflective surface 2a in the light transmission member 21. For example, the light transmission member 21 may be a prism, and light incident along one of the first optical axis A1 and the second optical axis A2 may be refracted on the surface 25b of the reflective portion 25 and thus reflected along the other of the first optical axis A1 and the second optical axis A2. Further, the light reflection unit 2 may not include the metal layer 22 and the protective layer 23, and the pushing surface 61a of the pushing member 61 may push the light reflection unit 2 to the X2 side while being in surface contact with the surface 25b of the reflective portion 25. According to this optical probe 1A (1B) of the first modification example, reflecting light, which is incident along one of the first optical axis A1 and the second optical axis A2, along the other of the first optical axis A1 and the second optical axis A2 can be realized with a simple configuration.

In the optical probe 1A (1B) of the first modification example, the pushing surface 61a of the adjustment unit 6 may be a rough surface. "The pushing surface 61a is a rough surface" means that the pushing surface 61a is a surface having a surface roughness of 20 µm or more. For example, the pushing surface 61a may be a surface having an arithmetic average roughness Ra of 25 µm or more, or may be a so-called rough-finished surface. In a case where the pushing surface 61a is a rough surface, the surface 25b includes an interface with air, for example, when the pushing surface 61a is in contact with the surface 25b of the reflective portion 25. According to this optical probe 1A (1B) of the first modification example, a sufficient difference in refractive index can be ensured on the surface 25b of the reflective portion 25, so that light incident along one of the first optical axis A1 and the second optical axis A2 can be reliably reflected along the other of the first optical axis A1 and the second optical axis A2.

In the above-described embodiment, the adjustment unit 6 only needs to be capable of adjusting the position of the light reflection unit 2 in the X-axis direction by pushing the light reflection unit 2 to the X2 side in the X-axis direction. For example, in an optical probe 1A (1B) of a second modification example, the screw member 62 may be movable forward and backward in the X-axis direction, and one end 62a of the screw member 62 may be in contact with the surface 61b of the pushing member 61. In the optical probe 1A (1B) of the second modification example, in a case where the other end 62b of the screw member 62 is rotated in the normal direction and the screw member 62 is moved forward, the light reflection unit 2 slides to the X2 side as in the above-described embodiment as the pushing member 61 is caused to slide to the X2 side by the pushing force of the screw member 62. On the other hand, in a case where the other end 62b of the screw member 62 is rotated in the reverse direction and the screw member 62 is moved backward, the pushing member 61 slides to the X1 side in the X-axis direction as in the above-described embodiment.

Further, for example, in an optical probe 1A (1B) of a third modification example, the adjustment unit 6 may include only the screw member that is attached to the housing 3 to be movable forward and backward along the X-axis direction. In this case, the optical probe 1A (1B) may not include the second biasing member 5; for example, the screw member may be attached to the wall portion 32. One end of the screw member in the X-axis direction may be in contact with the surface 24b of the support portion 24 of the light reflection unit 2, and the other end of the screw member in the X-axis direction may be exposed to the outside of the housing 3. According to this optical probe 1A (1B) of the third modification example, the adjustment of the position of the light reflection unit 2 in the X-axis direction can be realized with a simple configuration.

In the optical probe 1A (1B) of the third modification example, in a case where the other end of the screw member is rotated in the normal direction and the screw member is moved forward to the X2 side in the X-axis direction, the light reflection unit 2 slides to the X2 side as the surface 24b of the support portion 24 is pushed with the pushing force of the screw member by one end of the screw member. On the other hand, in a case where the other end of the screw member is rotated in the reverse direction and the screw member is moved backward to the X1 side in the X-axis direction, the light reflection unit 2 slides to the X1 side due to the biasing force of the first biasing member 4.

Since the position of the light reflection unit 2 in the X-axis direction only needs to be capable of being adjusted in the above-described embodiment and each modification example, the light reflection unit 2 only needs to be pushed to the X2 side in the X-axis direction by the adjustment unit 6 and only needs to be biased to the X1 side in the X-axis direction by the first biasing member 4. Therefore, the optical probe 1A may not include the second biasing member 5.

In the above-described embodiment and each modification example, one end 62a of the screw member 62 in the Z-axis direction only needs to be in contact with the pushing member 61. Therefore, the recessed portion 61f may not be formed in the pushing member 61.

In the above-described embodiment and each modification example, the screw member 62 only needs to be any screw member that is attached to the housing 3 and is movable forward and backward in a predetermined direction. For example, the screw member 62 may be a set screw.

The optical probe unit 10 includes one optical probe 1A and one optical probe 1B in the above-described embodiment and each modification example, but only needs to include at least one optical probe 1A and at least one optical probe 1B.

In the optical probe unit 10, one optical probe of the pair of optical probes 1A and 1B may be disposed on a straight line including the first optical axis A1 of the other optical probe of the pair of optical probes 1A and 1B. In this case, as the position of the light reflection unit 2 in the direction parallel to the first optical axis A1 is adjusted in the other optical probe, a distance between the second optical axis A2 of the optical probe 1A and the second optical axis A2 of the optical probe 1B can be adjusted according to the depth of a site at which optical properties are to be measured.

### Reference Signs List

1A, 1B: optical probe
2: light reflection unit
2a: reflective surface
2b: inclined surface
3: housing
4: first biasing member
5: second biasing member
6: adjustment unit
10: optical probe unit
11: holder
21: light transmission member
22: metal layer
23: protective layer
61: pushing member
61a: pushing surface
61f: recessed portion
62: screw member
62a: one end
62b: other end
121, 122: optical fiber
121a, 122a: end
A1: first optical axis
A2: second optical axis

## Claims

1. An optical probe comprising:
a light reflection unit comprising a reflective surface configured to reflect light, which is incident along one of a first optical axis and a second optical axis intersecting each other, along the other of the first optical axis and the second optical axis;
a housing that accommodates the light reflection unit such that the light reflection unit is slidable in a first direction parallel to the first optical axis and is configured to hold an end of an optical fiber such that the end is positioned on the first optical axis and faces the light reflection unit in the first direction;
a first biasing member that biases the light reflection unit to a first side in the first direction in the housing; and
an adjustment unit that adjusts a position of the light reflection unit in the first direction by pushing the light reflection unit to a second side opposite to the first side in the first direction in the housing.

2. The optical probe according to claim 1,
wherein the light reflection unit comprises an inclined surface corresponding to the reflective surface,
the adjustment unit comprises a pushing member comprising a pushing surface that is in contact with the inclined surface, and
the housing accommodates the pushing member such that the pushing member is slidable in a second direction parallel to the second optical axis.

3. The optical probe according to claim 2,
wherein the adjustment unit further comprises a screw member that is attached to the housing to be movable forward and backward along the second direction,
one end of the screw member in the second direction is in contact with the pushing member, and
the other end of the screw member in the second direction is exposed to the outside of the housing.

4. The optical probe according to claim 3,
wherein a recessed portion in which the one end of the screw member is disposed is formed in the pushing member.

5. The optical probe according to any one of claims 2 to 4,
wherein the light reflection unit comprises a light transmission member and a metal layer that is disposed on a surface of the light transmission member, and
the reflective surface is a surface of the metal layer facing the light transmission member.

6. The optical probe according to claim 5,
wherein the light reflection unit further comprises a protective layer that is disposed on a surface of the metal layer opposite to the light transmission member, and
the inclined surface is a surface of the protective layer opposite to the metal layer.

7. The optical probe according to any one of claims 2 to 4,
wherein the light reflection unit comprises a light transmission member, and
the light transmission member comprises the reflective surface and the inclined surface as the same surface.

8. The optical probe according to claim 7,
wherein the pushing surface is a rough surface.

9. The optical probe according to any one of claims 1 to 8, further comprising:
a second biasing member that biases the light reflection unit to the second side in the first direction in the housing.

10. The optical probe according to claim 9,
wherein a biasing force of the first biasing member is larger than a biasing force of the second biasing member.

11. The optical probe according to claim 1,
wherein the adjustment unit further comprises a screw member that is attached to the housing to be movable forward and backward along the first direction,
one end of the screw member in the first direction is in contact with the light reflection unit, and
the other end of the screw member in the first direction is exposed to the outside of the housing.

12. An optical probe unit comprising:
a plurality of optical probes, each of which is the optical probe according to any one of claims 1 to 11; and
a holder that holds the plurality of optical probes.
